# EUROPEAN PATENT APPLICATION

(11) **EP 3 459 807 A1**
(43) Date of publication of application: **27.03.2019**
(21) Application number: 18195738.2
(22) Date of filing: 20.09.2018
(51) Int. Cl.: B60W 40/08, B60W 50/16, B60W 30/14, B60W 50/14

(54) **SYSTEM FOR MONITORING AN OPERATOR**

(30) Priority: 22.09.2017 US 201762562130 P; 11.07.2018 US 201816032558
(71) Applicant: Aurora Flight Sciences Corporation, Manassas, VA 20110 (US)
(72) Inventor: CHUA, Zarrin Khiang-Huey, Manassas, VA 20110 (US); RYAN, Jason Christopher, Manassas, VA 20110 (US); KALGHATGI, Roshan, Manassas, VA 20110 (US); CHOI, Jae-Woo, Manassas, VA 20110 (US)
(74) Representative: Morrall, Jonathan Ian McLachlan

(57) **Abstract**

An operator monitoring system for use in a ground based vehicle is provided. The operator system includes a monitoring system to collect information regarding one or more characteristics of the operator during operation of the vehicle, a core platform configured to determine whether the one or more characteristics corresponds to a fatigue indicator, a response system configured to generate a warning based at least in part on the fatigue indicator and an interface to present the warning to the operator.

## Description

### CROSS-REFERENCE

The present application claims the benefit under 35 U.S.C. §119(e) of U.S. Provisional Patent Application Serial No. 62/562,130, filed September 22, 2017, and titled "System for Monitoring an Operator," the contents of which are hereby incorporated by reference.

### TECHNICAL FIELD

The present disclosure relates to vehicle-based operator monitoring systems, methods, and apparatuses. In particular, systems, methods, and apparatuses capture information regarding the operator's physical and/or physiological characteristics, analyze the information, determine a level of operator fatigue or health state, and/or provide warnings based at least in part on the information.

### BACKGROUND

Degraded performance due to fatigue or medical conditions is a contributor to most major accidents during operation of heavy machinery and vehicles, such as trains, automobiles, aircraft, boats, etc. Due to lack of focus, operators can miss external signals, misunderstand the impact of dynamic events, and/or fall asleep for periods of time during vehicle operation, resulting in reduced situational awareness. Operators experiencing fatigue put the operator, vehicle passengers, and the environment in which the vehicle is operating at risk, such as from collisions and other accidents.

Fatigue monitoring systems account for one or both physical and mental fatigue, by monitoring body characteristics and measurable human-machine interaction. Furthermore, operators may experience incapacitation due to medical conditions, such as hypoxia, heart failure, seizures, etc. Such incapacitation would place the operator, crewmembers, passengers, people and property in the area in which the vehicle operates, and the vehicle itself, in grave risk of collision or other damage. Thus, a system that is capable of monitoring and addressing fatigue or medical incapacitation during operation of a vehicle is desirable.

### SUMMARY

The present disclosure is directed to vehicle control systems, methods, and apparatuses; even more particularly, to a system, method, and apparatus to capture information regarding the operator's physical and/or physiological characteristics, analyze the information, determine a level of operator fatigue, and/or provide warnings based at least in part on the information.

In certain aspects, a system to monitor an operator of a vehicle is disclosed. The system includes a sensor to collect information regarding one or more characteristics of the operator. A core platform configured to determine whether the one or more characteristics corresponds to a fatigue indicator. A response system configured to generate a response (e.g. warning, mechanical, or cognitive intervention) based at least in part on the fatigue indicator. And an interface to present the response to the operator. In some examples, the characteristic corresponds to a physiological characteristic, and the sensor includes a physiological sensor to measure the physiological characteristic. The physiological characteristic is one of a heart rate, a respiratory rate, a blood oxygen level, or a body temperature.

In some aspects, the system includes a library of physiological characteristic values, wherein the change is determined by a comparison of a measured physiological characteristic value against a corresponding stored physiological characteristic value. The system also includes a classification system to identify an operator condition based at least in part on the comparison, the measured physiological characteristic value, and the stored physiological characteristic value. In some examples, the classification system includes one or more thresholds corresponding to the operator condition, wherein the operator condition includes awake, fatigued, and asleep.

In some aspects of the disclosure, the characteristic corresponds to a physical movement, which is one of a change in head position and/or orientation, a delayed reaction time, and a change in body position and/or orientation. The core platform further includes a library of physical movement values, with the change being determined by a comparison of a measured physical movement value against a corresponding stored physical movement value. The sensor is one or more of a visual camera, an infrared camera, a laser sensor, an ultrasound sensor, a temperature sensor, and/or a force sensor.

In an example, the interface provides the response aurally, visually, and/or by haptic feedback, and includes a touch screen display or other mechanical intervention, such as robotic actuation. Also included is a communication interface to connect to a network, the core platform to transmit another warning to a remote system via the communication system.

In additional or alternative aspects, a method of monitoring an operator of a vehicle is provided. The method includes sensing, via a plurality of sensors, one or more characteristics of the operator. The method determines, by a core platform, whether the one or more characteristics corresponds to a fatigue indicator, generates, by a response system, a warning based at least in part on the fatigue indicator, and presents the warning to the operator via an interface.

The method also includes identifying, by a classification system, an operator condition based at least in part on the measured physiological characteristic value, and the stored physiological characteristic value, applying, via the classification system, one or more thresholds corresponding to the operator condition. In some examples, the operator condition includes awake, fatigued, and asleep.

In certain aspects, the method includes determining, via the classification system, that the operator condition corresponds to being asleep, generating, via a command system, a command to control one or more vehicle functions in response to the asleep determination, and controlling one or more vehicle functions in response to the command. The one or more characteristics can correspond to a physiological characteristic, with the plurality of sensors comprising a physiological sensor to measure the physiological characteristic.

Additionally or alternatively, the method can include comparing a measured physiological characteristic value against a corresponding stored physiological characteristic value, applying one or more thresholds to the comparison, and determining an operator condition based at least in part on the comparison, wherein the operator condition includes awake, fatigued, and asleep.

As will be discussed, the operator monitoring system can provide significant benefits to a variety of end-users in a variety of industries. An example application includes the operation of vehicle where fatigue and boredom can cause a reduction in crew attentiveness, in which case the operator monitoring system reduces risk in a vehicle operation by alerting the operator and, in certain instances, assuming control of the vehicle. Other example applications exist where the potential for human error currently limits extensive use of vehicle, and improved debrief capabilities due to comprehensive data logging.

According to a first aspect, a system to monitor an operator of a vehicle such as a locomotive, an automobile, a spacecraft, an aircraft, a boat, a motorcycle or other form of transportation comprises: a sensor to collect information regarding one or more characteristics of the operator during operation of the locomotive; a core platform configured to determine whether the one or more characteristics corresponds to a fatigue indicator; a response system configured to generate a warning based at least in part on the fatigue indicator; and an interface to present the warning to the operator.

In certain aspects, the characteristic corresponds to a physiological characteristic, the sensor comprising a physiological sensor to measure the physiological characteristic.

In certain aspects, the physiological characteristic is one of a heart rate, a respiratory rate, a blood oxygen level, and a body temperature.

In certain aspects, the system further comprises a library of physiological characteristic values, wherein the change is determined by a comparison of a measured physiological characteristic value against a corresponding stored physiological characteristic value.

In certain aspects, the system further comprises a classification system to identify an operator condition based at least in part on the comparison, the measured physiological characteristic value, and the stored physiological characteristic value.

In certain aspects, the classification system comprises one or more thresholds corresponding to the operator condition, wherein the operator condition includes awake, fatigued, and asleep.

In certain aspects, the characteristic corresponds to at least one of (1) a change in head position or orientation, (2) a delayed reaction time, (3) a facial movement, or (4) a change in body position or orientation.

In certain aspects, the core platform is operatively coupled with a library of historical data associated with the operator and is configured to identify the fatigue indicator through trend analysis of the historical data.

In certain aspects, the core platform uses one or more machine learning algorithms to generate a library of expected operator actions or ideal operator actions for the locomotive, wherein the library is used to identify whether the one or more characteristics corresponds are associated with a fatigue indicator.

In certain aspects, the core platform further comprising a library of physical movement values, wherein the change is determined by a comparison of a measured physical movement value against a corresponding stored physical movement value.

In certain aspects, the sensor is one of a visual camera, an infrared camera, a laser sensor, an ultrasound sensor, a temperature sensor, or a force sensor.

In certain aspects, the system further comprises a communication interface to connect to a network, the core platform to transmit another warning to a remote system via the communication system.

According to a second aspect, a method of monitoring an operator of a vehicle comprises: sensing, via a plurality of sensors, one or more characteristics of the operator; determining, by a core platform, whether the one or more characteristics corresponds to a fatigue indicator; generating, by a response system, a warning based at least in part on the fatigue indicator; and presenting the warning to the operator via an interface.

In certain aspects, the method further comprises the step of identifying, by a classification system, an operator condition based at least in part on the measured physiological characteristic value, and the stored physiological characteristic value.

In certain aspects, the method further comprises the step of applying, via the classification system, one or more thresholds corresponding to the operator condition.

In certain aspects, the operator condition is at least one of awake, fatigued, or asleep.

In certain aspects, the method further comprises the steps of: determining, via the classification system, that the operator condition corresponds to being asleep; generating, via a command system, a command to control one or more vehicle functions in response to the asleep determination; and controlling one or more vehicle functions in response to the command.

In certain aspects, the one or more characteristics correspond to a physiological characteristic, the plurality of sensors comprising a physiological sensor to measure the physiological characteristic.

In certain aspects, the method further comprises the steps of: comparing a measured physiological characteristic value against a corresponding stored physiological characteristic value; applying one or more thresholds to the comparison; and determining an operator condition based at least in part on the comparison, wherein the operator condition is at least one of awake, fatigued, or asleep. In certain aspects, the method further comprises the step of assuming control or adjusting an operation of the locomotive based at least in part on the fatigue indicator.

An embodiment comprises a system to monitor an operator of a locomotive, the system that includes a sensor to collect information regarding one or more characteristics of the operator during operation of the locomotive; a core platform configured to determine whether the one or more characteristics corresponds to a fatigue indicator; a response system configured to generate a warning based at least in part on the fatigue indicator; and an interface to present the warning to the operator. The characteristic may correspond to a physiological characteristic, and the sensor may include a physiological sensor to measure the physiological characteristic. This will enhance operation. The physiological characteristic may be one of a heart rate, a respiratory rate, a blood oxygen level, and a body temperature. The system may also include a library of physiological characteristic values, wherein the change is determined by a comparison of a measured physiological characteristic value against a corresponding stored physiological characteristic value. The system may also include a classification system to identify an operator condition based at least in part on the comparison, the measured physiological characteristic value, and the stored physiological characteristic value. The classification system may include one or more thresholds corresponding to the operator condition, wherein the operator condition includes awake, fatigued, and asleep. The characteristic may correspond to at least one of (1) a change in head position or orientation, (2) a delayed reaction time, (3) a facial movement, or (4) a change in body position or orientation. The core platform may be operatively coupled with a library of historical data associated with the operator and is configured to identify the fatigue indicator through trend analysis of the historical data. The core platform may use one or more machine learning algorithms to generate a library of expected operator actions or ideal operator actions for the locomotive, wherein the library is used to identify whether the one or more characteristics corresponds are associated with a fatigue indicator. The core platform may also include a library of physical movement values, wherein the change is determined by a comparison of a measured physical movement value against a corresponding stored physical movement value. The sensor may be one of a visual camera, an infrared camera, a laser sensor, an ultrasound sensor, a temperature sensor, or a force sensor. The system may also include a communication interface to connect to a network, the core platform to transmit another warning to a remote system via the communication system.

Another embodiment comprises a method of monitoring an operator of a vehicle, the method that includes sensing, via a plurality of sensors, one or more characteristics of the operator; determining, by a core platform, whether the one or more characteristics corresponds to a fatigue indicator; generating, by a response system, a warning based at least in part on the fatigue indicator; and presenting the warning to the operator via an interface. The method may also include the step of identifying, by a classification system, an operator condition based at least in part on the measured physiological characteristic value, and the stored physiological characteristic value. This can enhance operation. The method may also include the step of applying, via the classification system, one or more thresholds corresponding to the operator condition. As a result, operation of this method can improve. The operator condition may be at least one of awake, fatigued, or asleep. The method may also include the steps of: determining, via the classification system, that the operator condition corresponds to being asleep; generating, via a command system, a command to control one or more vehicle functions in response to the asleep determination; and controlling one or more vehicle functions in response to the command. The one or more characteristics may correspond to a physiological characteristic, the plurality of sensors comprising a physiological sensor to measure the physiological characteristic. The method may also include the steps of: comparing a measured physiological characteristic value against a corresponding stored physiological characteristic value; applying one or more thresholds to the comparison; and determining an operator condition based at least in part on the comparison, wherein the operator condition is at least one of awake, fatigued, or asleep. The method may also include the step of assuming control or adjusting an operation of the locomotive based at least in part on the fatigue indicator.

### DESCRIPTION OF THE DRAWINGS

These and other advantages of the presently described systems, methods and apparatuses may be readily understood with reference to the following specification and attached drawings, wherein:
Figure 1a illustrates a block diagram of an example operator monitoring system.
Figure 1b illustrates an example flow of information data between the subsystems of Figure 1a.
Figure 2 illustrates a diagram of an example core platform architecture.
Figure 3 illustrates a block diagram of an example monitoring system.
Figure 4 illustrates an example method of implementing an operator monitoring system.

### DETAILED DESCRIPTION

Preferred embodiments may be described herein below with reference to the accompanying drawings. In the following description, well-known functions or constructions are not described in detail because they may obscure the subject matter in unnecessary detail. For this disclosure, the following terms and definitions shall apply.

Monitoring human-machine interaction provides additional insight as to the operator's performance, which correlates with fatigue. Any such interaction can be directly measured by connecting to any existing data bus and/or indirectly measured using cameras or other sensors that passively monitor the state of switches, gauges, throttles, etc.

The presently described system has been pioneered by Aurora Flight Sciences' in a Monitoring Engineer Fatigue (MEFA) system. The MEFA is an in-cab, passive monitoring system capable of detecting and/or intervening when a vehicle operator (*e.g*., a locomotive engineer) is determined to be less attentive during operation of the vehicle due to fatigue and/or health conditions. The monitoring system relies on one or more operator physiological and/or behavioral characteristics to infer the operator's level of fatigue. These characteristics come from multiple sources and are measured using sensors and a learned and/or calculated value associated with the operator's activity in the cab.

The MEFA system captures, synthesizes and analyzes data from multiple sources and/or multiple subjects, such as operator movements (*e.g*., eyes, head, body, etc.), and cab activity (*e.g*., operator responses and/or actions in view of controls). Analysis of multiple characteristics provides redundancy and creates confidence in the accuracy of the fatigue classification. Furthermore, independent characteristic sources increase the robustness of the system to various working conditions that conventional fatigue monitoring techniques (i.e., eye trackers) cannot accurately determine, such as extreme lighting conditions (*e.g*., very low and/or very hi levels of illumination), headwear (*e.g*., hats, helmets, etc.), eyeglasses and/or goggles, excessive movement of the operator and/or vehicle, etc.

The presently disclosed monitoring system overcomes these issues by being characteristic-dependent and sensor-independent, such that, as sensing technology improves, the sensors themselves can be upgraded and incorporated with the existing system architecture. Information from a variety of sensors is used to provide a subset of fatigue characteristics, such as visual cameras to register operator movements. Furthermore, multi-modal fatigue intervention techniques can quickly rouse the engineer from a non-vigilant state, or direct the operator's attention to the correct task actions.

Aurora has demonstrated vision-based cockpit system monitoring using a digital data bus. Cameras are mounted in a manner to minimize obstacles and obscurants in the operator workspace, line-of-sight visibility of all relevant panels and indicators, and/or to minimize operator body occlusion.

Within the workspace, operator performance can be gauged by comparing current physical and/or physiological characteristics against stored, expected characteristics. Inappropriate system engagement and/or delayed reaction times determined via the comparison can represent poor performance. For example, Aurora's Aircrew in Labor In-Cockpit Automation System (ALIAS) Knowledge Acquisition module is configured to digitize standard operating procedures, using trend analysis and/or training movements, a library or matrix of values corresponding to standard procedures can be downloaded and/or built, and used to determine task dependencies and parallels. The outputs of the ALIAS module can also be used to inform electronic checklists, moving maps, adjust heads-up displays, and/or provide text-to-speech reminders. Motion tracking of arm movements is also used as indicators of operator activity, providing a layer of redundancy if sensors, such as cameras, do not have an unobstructed view of the control panel. The range of reaction times of an operator in response to dynamic operational conditions, and can be approximated using first order models such as Fitts' law, with repeated usage values updated and stored in the library or matrix, and used to draw the comparison for future actions. ALIAS and other monitoring systems are described in greater detail by commonly own U.S. Patent Publication No. 2017/0277185A1 to Jessica E. Duda et al., titled "Aircrew Automation System and Method" and U.S. Patent Application No. 15/624,139 to William Bosworth et al., titled "System and Method for Performing an Emergency Descent and Landing."

A variety of vehicle types, work and operating environments, as well as operators can benefit from the described monitoring system. For example, operators in the rail industry and long-distance trucking face challenges such as long shifts and monotonous scenery. Further, the aerospace and naval industries often operate in challenging conditions that require near complete operator attention. Commonplace automobile operators can similarly benefit from the system as well.

In an example, monitoring the operator's eye can offer characteristics for identifying operator fatigue (*e.g*., via analysis of the operator's percentage of eye closure, or "PERCLOS"). For example, the movement and state of the eye are measured using a fixed or head-mounted eye tracker. Eye trackers can also provide the direction of the operator's gaze. For instance, prolonged lateral gazing in a forward-facing activity such as operating a vehicle is an indicator of low vigilance and possibly fatigue. In some examples, occlusion of the eye, such as from the use of glasses or sunglasses, is mediated by monitoring other characteristics.

Head and body dynamics provide additional or alternative characteristics of operator fatigue. For example, head drooping (*e.g*., nodding off) and off-axis body positions (*e.g*. off-center, reclining, slumped shoulders) typically occur at the onset of sleepiness. Conversely, fatigued operators may lean against objects within the operating environment and/or prop up the head with an arm. Motion tracking sensors are capable of detecting such head and body movements.

Medical conditions such as heart failure can be a precursor to full incapacitation and can be an indicated by actions by the operator, such as coughing and/or wheezing, and/or physiological characteristics, such as an increased heart rate. At the onset of a heart attack or cardiac arrest, the head may move to an unnatural orientation, with eyes closed.

In another example, hypoxia is defined as a shortage of oxygen in the blood to the brain. For instance, pilots operating an aircraft at high altitudes (*e.g*., above 8,000 feet) are at risk of hypoxia, with severity of the condition increasing proportionally to the aircraft altitude, although individuals may demonstrate these and/or other symptoms at different times. Full incapacitation brought on by hypoxia can have symptoms similar to a heart attack or cardiac arrest, such as head drooping and closed eyes. Seizures, on the other hand, are characterized by jerking motions of the head, eyes, and body. During onset of a seizure, heart rate changes rapidly, where some individuals may demonstrate either a lower or a higher heart rate compared to the operator's normal rate.

In a particular example from industry, rail operator fatigue is a major problem. For example, operators work long shifts with few breaks, and operation of a locomotive of train can often be monotonous (*e.g*., hours of nighttime travel, long stretches with little scenery, etc.). Thus, frequent fatigue and boredom results in missed items along the path of travel, such as railway wayside signals. Such signals/signage are critical for safe operation of the rail industry, as they instruct the operator to stop, slow down, be aware of changing track conditions, hazards on the way, etc. Similar issues can arise on roadways, as long-haul truckers and car operators also miss or fail to react to stop signs, signals, etc.

Degraded performance due to fatigue is a contributor to accidents in a variety of industries outside of rail, such as long-distance hauling. Vehicle operators may miss wayside signals or other relevant cues and/or information, because of reduced situational awareness and/or the effects of fatigue and/or health issues while operating the vehicle. Operators plagued by fatigue or other issues put him or herself at risk of an accident, including passengers and areas in which the vehicle operates.

Some alerter systems attempt to maintain operator alertness, existing alerter systems do not account for whether the engineer is mentally engaged in operation of the vehicle. As such, some alerter systems deactivate upon any operator interaction with a control of the vehicle system. For instance, an operator may be awake enough to press a particular button (e.g., via muscle memory), yet be fatigued to a level where situational awareness of their surroundings and/or the operation is impaired.

The federal railway association (FRA) has long studied ways of combating fatigue in the railroad industry. The FRA is interested in research and projects that address the railroad industry's susceptibility to the risk of injury and property damage caused by human fatigue and loss of attentiveness. This susceptibility is the result of several inevitable factors, such as around-the-clock operations, solitary work environments, uninspiring scenery, and other issues faced by railroad operators. Several features regarding the work and activities of an operator have been studied, including the impact of the following on a vehicle operator: Scheduling/calling systems for operators; shiftwork; calling assignments; lodging conditions; commute times; sleep disorder screening and treatment; fatigue education; the effectiveness of fail-safe technologies; and others. Unfortunately, common results are irregular work hours, long shifts, and an unpredictable schedule. The FRA seeks interventions or solutions to mitigate such effects.

In a particularly tragic example, a deadly accident occurred in Macdona, Texas, in 2004 (NTSB/RAR-06/03) involving an engineer that was able to demonstrate automatic behavior but not true attentiveness. In other words, the engineer was mentally fatigued and experiencing degraded performance, but was physically awake enough to continue providing input to the locomotive control system (*e.g*., automatic response to according to a learned behavior). This is relevant, as motor reflex responses typically require lower level cognitive effort. Thus, the operator was able to operate the locomotive despite his impairment; the engineer's actuation of a button or control served to reset the alerter system, which did not trigger to rouse the engineer to a more alert state.

However, the accident investigation found that the engineer's interactions with the vehicle controls were inappropriate given context of the immediate task. In particular, the engineer had increased the speed of the locomotive when the speed should have been decreasing. The presently disclosed monitoring system is configured to generate alerts in response to unexpected and/or improper operator interactions, such as engaging the throttle in the wrong direction.

Situations and conditions still exist that require attention, such as areas of low illumination, or rail line parts (*e.g*., grade crossings) that have not been incorporated into other systems (*e.g*., due to expensive infrastructure, complex networking, etc.). In these and other areas of limited coverage, even a captured situation may require immediate human intervention.

The system itself is also designed to be characteristic-dependent and/or sensor-independent, meaning that as sensing modalities and/or motion tracking technologies develop, such equipment can be integrated with an existing system architecture.

Thus, the monitoring system described herein provides a consistent, reliable, and accurate detection and/or intervention of fatigue and/or health conditions. As the monitoring system can be implemented as a standalone system, wide industry acceptance is expected. Further, increased functionality is offered if the system is paired with road and/or rail autonomy aids, such as a heads-up display, external perception, GPS, etc.

In an effort to mitigate these and other potentially catastrophic events, the system described herein provides a plurality of sensors to capture data corresponding to one or more operator characteristics, and a core platform configured to analyze the data by employing "deep learning" or "machine learning" techniques to determine the operator's condition therefrom.

Data collected from optical capture systems (*e.g*., one or more types of cameras) can be integrated with other data collection sources (*e.g.,* physiological sensors, vehicle state sensors, stored information, etc.) for a more complete understating of the operator's condition. System responses will be accomplished through any number of modalities configured to arouse and/or otherwise engage with a fatigued operator, such as a human-machine interface (HMI) such as a tablet and/or computer screen, audio source, haptic feedback device, etc. The system is configured to prompt the operator to act in response to an alert, and/or confirm what action, if any, is to be taken.

In some examples, if the operator fails to provide an appropriate response, the system can be further configured to control one or more functions of the vehicle to address an identified hazard, such as automatically decelerating the vehicle, stopping the vehicle, and/or generating an external alert (*e*.*g*., to a remote command center, via a system perceptible to those near the vehicle and/or the vehicle path, etc.).

The system described herein is configured to operate in real-time via multiple modalities to identify and/or generate a response for a fatigued operator. By employing a computer assisted, vision enabled monitoring system that uses machine learning/deep learning techniques for capturing information associated with an operator, determining a condition of the operator, and/or generating a response to engage with the operator, the system is capable of avoiding potentially hazardous situations.

Information collected from the various sensors is compiled and analyzed as a whole, in view of stored data including historical trends, to quickly and accurately build a picture of an operator's expected and/or common condition. In other words, the core platform is configured to accept, analyze, and/or make determinations based at least in part on the various sensor information, or "sensor fusion", among sensors of differing types, such as visual sensors, physiological sensors, vehicle state sensors, to name but a few. Thus, machine learning/deep learning techniques, capable of collecting data and building models over time to recognize and adapt to similar situations in the future, are used to overcome limited views, damaged identifiers, variable lighting conditions, to name a few.

In a given implemented example of the described system, any number and type of human-machine interfaces can be present, from audio, visual and haptic sources, to systems to accept voice commands for automated "smart" systems, as well as conversion to text for another operator and/or system with access to a networked, visual monitoring system.

Aurora Flight Sciences Corporation of Manassas, Virginia has developed autopilot capabilities for flight-enabled vehicles. Aurora Flight Sciences has experience with machine vision systems in aircraft and machine learning from the Aircrew in Labor In-Cockpit Automation System (ALIAS) and Digital Flight Engineer (DFE) programs. Under these programs, Aurora developed a machine vision operator system to read and process the instruments in on an aircraft instrument panel with high enough fidelity to accurately derive the aircraft state and, in turn, automatically fly the aircraft using an onboard autopilot. This was demonstrated in five different cockpit types, three in flight across a variety of flight conditions. Aurora will leverage the lessons learned from these programs with respect to imaging hardware and software development to create an operator monitoring system. The innovation is in the application and refinement of the techniques for monitoring operator conditions based at least in part on captured information.

The rail industry has studied means to detect fatigue, primarily concentrating on eye tracking and wearable devices. However, no existing research or systems exploit activity monitoring to inform fatigue of operator fatigue levels. In one aspect, intelligent electronic checklists are employed as a method of ensuring complete system health, to be overseen by the operator. Such complementary and overlapping information capture and measurement capabilities provide a solution for shortcomings in other systems (*e.g*., complex and expensive equipment, lighting sensitivity).

This operator monitoring system provides a low-cost, robust, real-time response to operator fatigue. The system supplies a monitoring system with multiple safeguards that fill an area not currently addressed with existing alerter systems, such as when the operator is mentally disengaged from current tasks, but physically awake enough to nullify the effects from existing alerter systems. Thus, the present system provides a more accurate assessment of operator fatigue. When coupled with response system, the risk of accidents due to fatigue should be reduced.

Moreover, the operator monitoring system can be employed with autonomous vehicle operating system and/or external perception systems to enhance operation of complex platforms by increasing operator downtime, better allocating operator resources, and/or eliminating the need for reliance on human operators in certain tasks

As utilized herein the terms "circuits" and "circuitry" refer to physical electronic components (*i*.*e*. hardware) and any software and/or firmware ("code") which may configure the hardware, be executed by the hardware, and or otherwise be associated with the hardware. As used herein, for example, a particular processor and memory may comprise a first "circuit" when executing a first set of one or more lines of code and may comprise a second "circuit" when executing a second set of one or more lines of code.

As utilized herein, "and/or" means any one or more of the items in the list joined by "and/or". As an example, "x and/or y" means any element of the three-element set {(x), (y), (x, y)}. In other words, "x and/or y" means "one or both of x and y". As another example, "x, y, and/or z" means any element of the seven-element set {(x), (y), (z), (x, y), (x, z), (y, z), (x, y, z)}. In other words, "x, y and/or z" means "one or more of x, y and z". As utilized herein, the term "exemplary" means serving as a non-limiting example, instance, or illustration. As utilized herein, the terms "e.g.," and "for example" set off lists of one or more non-limiting examples, instances, or illustrations.

As used herein, the words "about" and "approximately," when used to modify or describe a value (or range of values), mean reasonably close to that value or range of values. Thus, the embodiments described herein are not limited to the recited values and ranges of values, but rather should include reasonably workable deviations. As utilized herein, circuitry or a device is "operable" to perform a function whenever the circuitry or device comprises the necessary hardware and code (if any is necessary) to perform the function, regardless of whether performance of the function is disabled, or not enabled (*e.g*., by a user-configurable setting, factory trim, etc.).

As used herein, the terms "communicate" and "communicating" refer to (1) transmitting, or otherwise conveying, data from a source to a destination, and/or (2) delivering data to a communications medium, system, channel, network, device, wire, cable, fiber, circuit, and/or link to be conveyed to a destination. The term "database" as used herein means an organized body of related data, regardless of the manner in which the data or the organized body thereof is represented. For example, the organized body of related data may be in the form of one or more of a table, a map, a grid, a packet, a datagram, a frame, a file, an e-mail, a message, a document, a report, a list, or data presented in any other form.

Disclosed herein is a system configured to, *inter alia*, monitor one or more conditions of an operator of a vehicle. Such an automated operator system may be configured to continuously monitor operator actions, expressions, responses, physiological data, etc. during travel, as well as automatically generating one or more warnings or alerts to the operator or other responsible party and/or system in response to determination of one of a variety of operator states (*e.g*., fatigue). Additionally or alternatively, the system is configured to control one or more vehicle subsystems associated with the vehicle based at least in part on such a determination.

In particular, one or more physical and/or physiological characteristics are monitored and analyzed, such as behavioral, neurological, and other conditions. A determination is made as to whether the operator's physical and/or physiological characteristics correspond to a potential fatigue situation or negative health condition, and an appropriate warning is generated in response. The system leverages a variety of characteristics from independent physiological and/or performance-based sources (*e.g*., a library or matrix of values and/or data) used to determine an operator's level of fatigue and/or health condition, and intervene if the level exceeds a threshold level. The physiological characteristics come from a variety of sensors configured to passively and/or non-invasively monitor the operator. The performance-based characteristics are inferred through human-machine interaction monitoring, including tracking the operator's movements.

In the context of railroad vehicle operations, the core platform 102 is configured to digitize information in accordance with the GCOR, utilizing machine-learning technology (*e.g*., artificial intelligence) and/or subject matter expert (SME) analyses to determine task dependencies and parallels, such as within the locomotive cab. In some examples, machine learning employs algorithms to generate a library of expected and/or ideal operator actions and/or movements in view of the specific vehicle being operated. The actions can be assigned any number of values associated with the operator action (*e.g*., speed, trajectory, contact with an instrument, etc.). Based on the values, the machine learning algorithms can build a profile and set thresholds and/or representative examples used to identify an action as being associated with a fatigue characteristic. Once a fatigue characteristic is identified, the values can be compared against one or more thresholds to determine the severity of the operator fatigue condition.

For example, thresholds can correspond to a low risk of loss of attention, which may generate a warning via the warning system 108a. A higher level threshold may correspond to an action to be taken, such as via the command system 108b. Further, a number of thresholds can be used, with an array of responses resulting therefrom. In some situations, the thresholds can correspond to an escalation of the responses, from non-invasive alerts to vehicle control (*e*.*g*., a visual warning, an aural warning, haptic feedback, request for an operator response, communication to a remote system, automatic control of a braking system, etc.). Additionally or alternatively, SMEs determine which tasks performed by the operator are impacted by operator fatigue, and how great the risk of an accident.

An affirmative determination of a fatigue and/or health classification may trigger an intervention (*e.g*., a warning, an alarm, etc.) to focus the engineer on the task of operating the vehicle. For example, multi-modal fatigue intervention techniques can quickly rouse the engineer from a micro-sleep state and/or redirect the operator's attention. The system is designed to respond to sensor input and characteristic classification; in other words, the type and sensitivity of the sensor, and the detail and volume of stored data, can vary such that upgrades and expanded reference material is incorporated into the system to provide updated responses, without reconfiguring or replacing the system. The sensors can include motion trackers, eye trackers, cameras, data buses, etc., and may be supplemented and/or substituted by other sensors, such as RGB, IR sensors, Electromyography (EMG), depending on the vehicle, operating environment, processing capacity, etc.

Due to the nature and variety of sensors, the operator monitoring system is customizable over a variety of vehicles. Thus, the operator monitoring system may be temporarily installed and/or readily transferred from vehicle to vehicle, without extensive modifications. The operator monitoring system, through its modular design, further reduces the likelihood of designing a single point solution that becomes obsolete as vehicles and operations evolve.

The operator monitoring system's combination of subsystems provides high-fidelity knowledge of the operator's physical state, and generates a response (*e.g*., a warning, alert, etc.) based on, for example, predictive models and/or information stored in a matrix of values corresponding to expected operator characteristics.

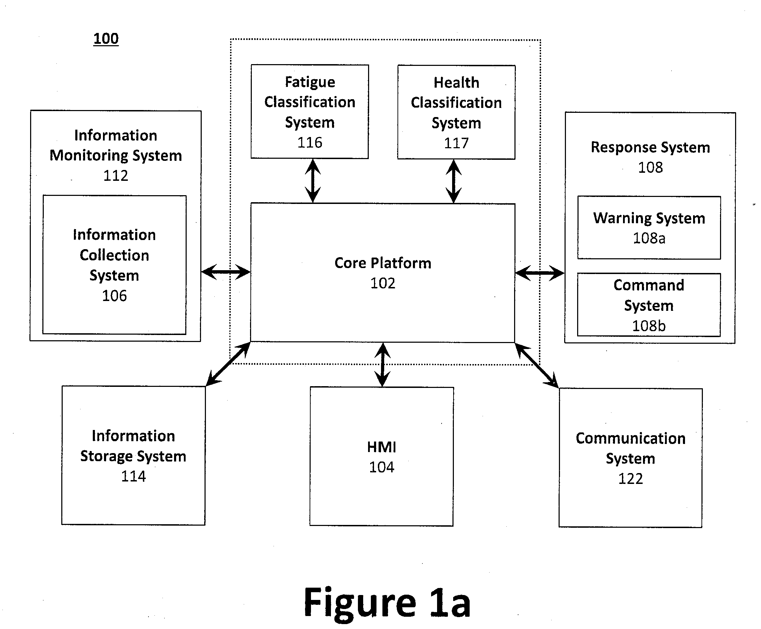

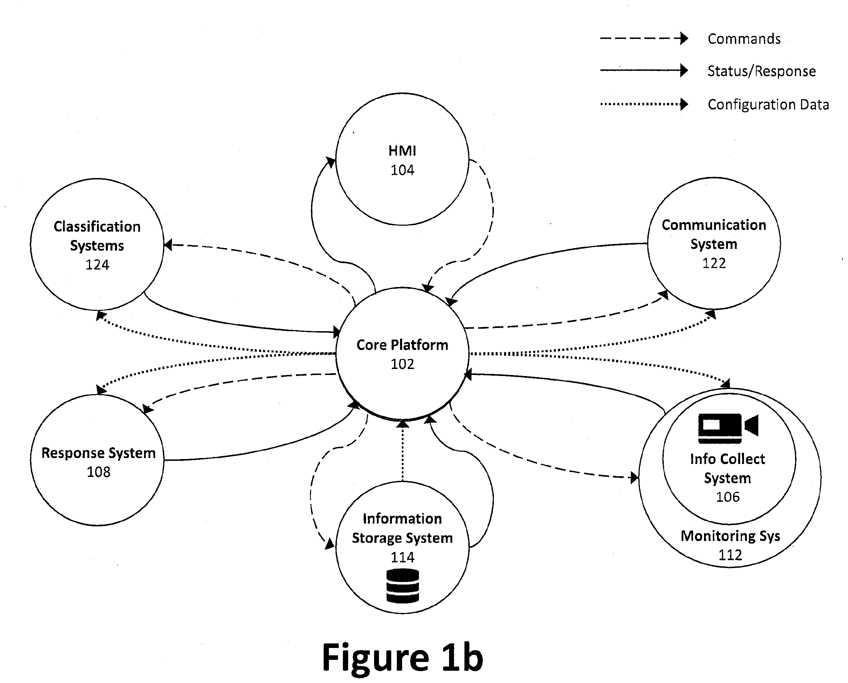

***System Level Architecture.*** An example system architecture for an operator monitoring system 100 in accordance with one aspect is shown in Figures 1a and 1b. The operator monitoring system 100 may be integrated with, or otherwise installed on, a vehicle (*e.g*., a locomotive). As illustrated in Figure 1a, the core platform 102 may operate as a central subsystem that connects other subsystems via one or more interfaces. The subsystems may communicate with one another through software and/or hardware interfaces using wired and/or wireless communication protocols and hardware. Figure 1b illustrates an example flow of information (*e.g*., data) between the various subsystems.

The plurality of subsystems may include, for example, the response system 108, the HMI system 104, fatigue classification system 116, and health classification system 117, each of which may be operatively coupled with the core platform 102. In certain aspects, in addition to data from the various sensors, information from the vehicle cab can be fed to the core platform 102 to aid in the learning and/or decision making process. For example, the operator monitoring system 100 may couple (*e.g*., communicatively or electronically) with the instrument panel, or be otherwise integrated with the vehicle or its systems to provide information regarding operator interaction with the vehicle which can correspond to operator movements and responses. As can be expected, however, such integration would likely require a degree of modification to the vehicle or its wiring. The operator monitoring system 100 and/or core platform 102 may also comprise, or be operatively coupled to, an information storage system 114 and a communication system 122.

In operation, the core platform 102 derives the vehicle state based on information data from another subsystem (*e*.*g*., information collection system 106) and directs another subsystem (*e.g*., the response system 108) to operate (*e.g*., dynamically) in a manner to maintain safe vehicle operation. For example, the vehicle may receive commands from the command system 108b, while sending to the core platform 102 information generated by the vehicle. In some examples, the system requires the operator to respond to certain stimuli. Such a system is effective in providing situational awareness to aid in prevention of various situations that could lead to accidents, such as a fatigued or sleeping operator or crewmember.

The system includes an information monitoring system 112, which includes an information collection system 106. Multiple sensors, including a plurality of cameras, aid in monitoring the condition of the operator and/or the state of the vehicle and/or conditions in the surrounding environment.

***Open Architecture**.* The core platform 102 serves as the central hub, or interface, of the operator monitoring system 100, connecting and controlling the remaining subsystems (*e.g*., as individual applications) in an open architecture. The remaining subsystems include, for instance, the HMI system 104, the response systems 108 (*e.g*., the warning system 108a and command system 108b to provide autonomous operation where desired), the information collection system 106, information storage system 114, and other subsystems 236. Thus, control of the other operator monitoring system 100 hardware may be provided via separate applications specific to a particular piece of hardware, which enables rapid integration of new systems or other external vehicle support technology.

The core platform is configured to incorporate and analyze data associated with multiple characteristics from different groups; an evaluation of operator task performance; use of vehicle state data; and interpretation of each characteristic in absolute and/or individualized terms. In other words, an absolute characteristic is common to all operators (*e.g*., if an operator's eyes are closed for an extended period, the system will determine an operator is sleeping), whereas some characteristics can be specific to the operator and/or vehicle operation (*e.g*., relative heart rate) as some individuals may demonstrate different responses and/or reactions to a similar stimulus. This system is extensible to different vehicle models and vehicle types (*e.g*., boats, cars, trucks, trains, aircraft, etc.) and can be coupled with other systems to improve the relevancy of the fatigue classification.

In an example, the core platform 102 communicates with one or both of the fatigue classification system 116 and the health classification system to derive specific values to classify the level of fatigue or health condition, respectively. For example, the information collection system 106 provides measured data corresponding to eye closure, which is compared to data corresponding to stored information associating eye closure rates and/or measure with degrees of fatigue. The fatigue classification system 116 compares the measured data to the stored data and calculates a value corresponding to the severity of the fatigue. If the fatigue is determined to be sufficiently severe (*e.g*., exceeding a warning threshold), the core platform 102 transmits the determination to the response system 108. In this example, the warning system 108a generates a warning for the operator, provided via the HMI 104, for instance.

Post-processing may be used to extract values corresponding to a given characteristic from the raw sensor data. For example, an RGB camera may give an indirect measure of heart rate that can be calculated based on visually captured differences between individual frames from video focused on the operator's body to extract the heart activity. In this example, the data is combined and time synchronized by the core processor 102, in order to determine the movements and which characteristic the movements represent. The collected data is used for "training" the fatigue classification system 116 to identify fatigue, in addition to determining thresholds to apply to characteristic data.

Training data for fatigue may be collected in real-time during operation of the vehicle by way of an initial calibration routine, or information may be compiled from long-term data from previous shifts. The calibration routine may consist of the operator striking various poses associated with fatigue to teach the system how to identify an individual's representative physical manifestation of fatigue (*e.g*., yawning, rubbing of eyes, gaze, linguistic changes, change in complexion, etc.). Similarly, reaction rates to various tasks may be calibrated based on human-machine interface exercises. During individual training, the characteristic classification algorithm results are also compared against standard sleepiness using scales such as the Karolinska Sleepiness Scale. In some examples, information collected from a different of many different operators can be compiled to generate a store of information related to fatigue (as well as health data, etc.). A range of acceptable characteristics and/or movements can be determined, and thresholds applied to classify the severity of such characteristics (*e.g*., prolonged eye closure).

The fatigue classification system 116 determines a state of the operator, such as whether the operator is awake, fatigued, or asleep. Combinations of various characteristics, or a subset of combinations, are used to provide a suitable data set upon which to base the determination. The fatigue classification system 116 outputs a final state assessment, including the confidence in the response. Once the operator fatigue state has been classified, asleep or fatigued states can be transmitted to the core platform 102 and or to the response system 108, which triggers a warning in the cockpit, cabin, etc., from the warning system 108a.

Data from the information collection system 106 and determinations from the fatigue classification system 116 (as well as health classification) are processed in real-time (*e.g*., collected, filtered, down sampling, applied to proper algorithms, etc.). For example, data from the information collection system 106 are synthesized to provide a set of operator characteristics per time unit for classification. Classification of the operator's state of fatigue uses machine learning algorithms (*e.g*., via fatigue classification system 116) such as support vector machines or artificial neural networks.

In some examples, the information collection system 106 is configured to translate operator movements (*e.g*., head and eye) to generalized geometric shapes that are used to determine position, trajectory, movement, speed, etc. Sensors employed by the information collection system 106 (*e.g*., cameras) are used for activity monitoring may be located behind the operator. The monitoring system leverages multiple characteristics determined based on independent physiological, biological and/or performance-based information sources to classify an operator's level of fatigue. Such a system uses sensors to passively and/or remotely monitor the operator. The fatigue classification system 116 is used to identify and/or trigger fatigue intervention methods to quickly re-engage the operator. In a similar manner, the health classification system 117 can intervene to address a pending or actual health condition.

In an example, three levels of classification are employed, having identified and stored information regarding the most frequently occurring characteristic combinations corresponding to a fatigue condition. As provided, supra, the levels can correspond to threshold values based on data stored in the information storage system.

A first level corresponds to the operator being asleep. In this example, the operator is physically and mentally disengaged, identified by such cues as a slumped head and body position, closed eyes, and/or a lack of interaction with controls, alerts, and/or other stimuli. A second level corresponds to the operator being fatigue. For instance, the operator is determined to be physically engaged, but mentally disengaged. The operator's head appears to be drooping, with eyes partially closed and/or locked in a non-forward gaze. The operator movements registrar limited movement or with a slower reaction time than expected and/or required by the system, or the interactions result in an incorrect end-state. A third level corresponds to the operator being awake. An awake operator is physically and mentally engaged. The head and body are erect, with eyes open, and correct interactions and/or reaction times registered in view of the expected tolerance.

The classification algorithm, levels, thresholds, etc., may be developed by employing one or more algorithms and/or with training data analyzed by SMEs. The system will be taught how to correctly interpret fatigue/health characteristics, and/or build a store of characteristics for comparison (*e.g*., at information storage system 114). The data will be collected through a series train operating activities, such as grade crossings, via real world or simulated events. In some situations, the simulations are completely computer conducted, such that a human operator is not used. The collected data will be divided for training the system (*e.g*., building comparison information) and testing of the classification algorithm (*e.g*., for classification purposes).

In some examples, data from one or more sensors can be weighted differently, based on the situation in which the operator and/or vehicle operates, particular characteristic of the individual operator, or other reasons designed to generate an accurate determination. For example, in an airplane, the operator (*e*.*g*., pilot) would be expected to increase in heart rate during ascent and descent. The core platform 102 is configured to recognize that the airplane is undergoing an intentional change in altitude, and weigh the operator's heart rate accordingly. Conversely, if the operator registers an unexpected quickening of heart rate, and that data is followed by measurements suggesting the airplane is experiencing an unintentional descent, the data can be used to classify the severity of the situation (*e*.*g*., which may lead to the command system 108b controlling one or more functions of the vehicle).

Once analyzed, the core platform 102 outputs an operator fatigue state (*e.g*., awake, fatigued, asleep, etc.). This classification can be transmitted to the response system 108 for intervention, which triggers a warning and/or a vehicle command. These alerts could be provided in conjunction with existing vehicle human-machine interfaces, such as control displays and/or vehicle speaker systems, and/or a dedicated device (*e.g*., a tablet computer) with audio, visual, text-to-speech capabilities.

The warning system 108a generates warnings, such as visual and audio warnings, which can include alerts tailored for the individual operator and/or situation (*e.g*. calling out the operator's name; directives such as "wake up!"). Stimulating music, dialogue, and/or other sources of entertainment, or recommendations for appropriate caffeine or other stimulants can be provided. Such warnings or alerts may be used in conjunction with other human-machine interfaces available to the operator, such as handheld tablets, cellphones, or heads-up displays.

In some examples, a tactile feedback device is used. A sudden and strong force on the body may provide redundancy in rousing the operator, although the intensity and localization of such vibrations must be carefully considered as to avoid being confounded with normal vehicle operation vibrations (*e.g*., the torso, wrist, feet, etc.). The fatigue classification system 116, when provided with data from context-based libraries, such as can be found in information storage system 114, can determine an acceptable napping period.

The health classification system 117 can apply characteristic thresholds to data corresponding to an operator's state of health. For example, measurements from one or more sensors can be used to determine one or more health conditions, such as hypoxia, seizure, heart failure, etc. The health classification system 117 may be calibrated to an individual operator's physical characteristics.

The health classification system 117 determines whether the operator is in any of a number of identified extreme health states (*e.g*., hypoxia, seizure, heart failure, etc.), which would require different interventions. For example, hypoxia can be mitigated by flying an aircraft to a lower altitude, whereas seizure and heart failure would result in immediate grounding of an aircraft. Similarly, seizure may require physically intervening with the operator as to minimize the risk of unintentional actions engaging the vehicle. In any such cases, automated robotic mechanisms (*e.g*., an autopilot, an arm or a series of smaller mechanisms) may be used to take control of a vehicle, and/or reprogram an autopilot system, and/or physically engage with the operator. In some examples, once a determination has been made that the operator is experiencing an extreme health condition, the response system 108 can generate a call for help, transmitted via the communication system 122. Additionally or alternatively, the command system 108b can serve as autopilot, or generate commands for a robotic mechanism, to operate a function of the vehicle to avoid a potentially hazardous situation. For example, if the operator of a road vehicle is experiencing a debilitating seizure, the command system 108b can decelerate the vehicle, turn on hazard lights, and/or direct the vehicle to the side of the roadway, to avoid a collision.

Given the severity of such health states, the HMI 104 can request a response from the operator to verify whether the operator is truly incapacitated. Such verification may exist in the form of verbal and/or tactile interaction. Thus, the operator may be asked to press a button on an interface, enter a code or password into a device, and/or respond within a set timeframe, make a particular hand gesture, and/or some combination of oral and verbal interaction, in order to cancel the classification.

Once operational, data associated with the fatigue and health classification schemes and responses thereto may be used to learn the effectiveness of the intervention system. Thus, best practices can be identified and enhanced, and ineffective or damaging interventions can be avoided. The health classification algorithm may also improve during the identification of false positives, for example, when the operator confirms with the algorithm that a given state has been incorrectly determined.

The core platform's 102 architecture enables rapid portability and extensibility when transitioning to a new vehicle or incorporating a new vehicle feature/capability. Thus, an application may be used to enable the operator monitoring system 100 to acquire information for that vehicle or to provide the new capability. For example, transition and setup can be handled by individual applications that operate within the core platform 102 or other subsystems, representing vehicle-specific functionalities as well as a growing library of capabilities of operator monitoring system 100, which can be exchanged depending on vehicle or crew requirements. In certain aspects, the transition process may be supported by software applications external to the operator monitoring system 100 (such as a procedure editor).

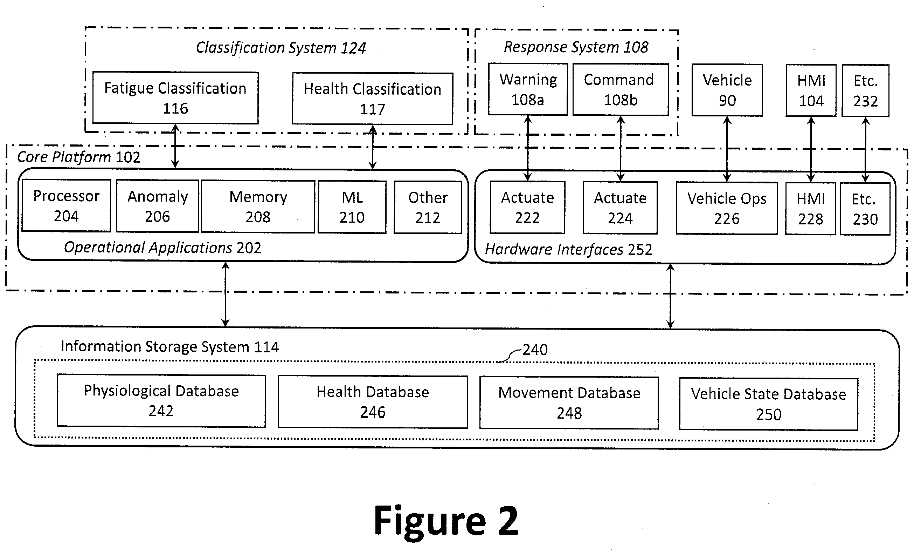

***Core Platform 102.*** Figure 2 illustrates an architecture diagram of an example core platform 102. To enable a vehicle-agnostic operator monitoring system 100, a core platform 102 may provide, or otherwise serve as, software, hardware, middleware, processing, etc., that can be made specific to a particular vehicle or configuration through an initial transition and setup phase. In other words, the core platform 102 provides an operating system that provides services to a set of operational applications 202 and output signals to one or more of a set of hardware interfaces 220, while collecting and logging the data necessary to enable those applications.

The monitoring system 100 is implemented by employing several components and/or modules, such as information monitoring system 112 to collect information via one or more sensors within an information collection system 106; an information storage system 114, configured to digitalize specific and general codes of operating rules (GCOR) or a Pilot Operating Handbook (POH), as well as capturing operator task dependencies and parallels. In some examples, the information collection system 106 determines the vehicle state (*e.g*., position of one or more controls and/or instruments, information from the vehicle operating system, etc.), as well as the operator characteristics, such as by use of video and audio sensing.

The ALIAS system is employed using minimally invasive techniques and equipment, allowing rapid extensibility and for modules to be adapted for other vehicles and/or operators (*e.g*., in the rail industry). The result is safety benefits and cost savings from increased operating efficiency by employing fail-safe technology (*e.g*., with layers of redundancy) that minimizes the number of accidents due to a fatigued state, such as when an operator is not fully awake but neither fully asleep. The system therefore addresses the operational gap that previous systems cannot due to their design limitations. Additionally or alternatively, the described monitoring system can capture data regarding a health condition of the operator, and analyze and determine a response to avoid a potentially dangerous operating situation, as described herein.

The core platform 102 serves as the primary autonomous agent and decision-maker, which synthesizes inputs from the information collection system 106 and HMI system 104 with its acquired knowledge base to determine the overall system state. The core platform 102 may process inputs from the various sensor suites and aggregate the information into an understanding of the vehicle's current operational state. The information may be compared against a vehicle specific file that encompasses the operator monitoring system's 100 understanding of operator intent, system health, and understanding of appropriate vehicle procedures as they relate to the operator monitoring system's 100 state estimation. The resultant state knowledge and associated recommendations can be passed to a human operator via the HMI system 104 or, in certain aspects, to the vehicle control system 124 and/or response system 108 to enable autonomous operation. In the example of Figure 1a, the response system 108 is connected to vehicle 90. Thus, a warning (via warning system 108a) and/or a command (via command system 108b) can be transmitted to the vehicle 90. This can include sending commands to one or more vehicle functions of the vehicle 90. Further, the operator monitoring system 100 may further generate a log of an operation for later analysis, which may be used to facilitate operator training. The logs may be used in connection with, for example, operational quality assurance analysis, maintenance analysis, etc.

***Response System 108**.* A response system 108 can process the information (*e.g*., identification, interpretation, relative position) to determine one or more actions to rouse or otherwise engage with the operator, such as a warning or other alert. The warning can be customized for the determined level of operator fatigue. For example, types of warnings can include, but are not limited to, visual alerts, audible alerts, haptic or vibrational feedback, transmission of alerts to multiple entities (*e.g*., other crewmembers, a remote monitoring station, etc.).

A response can be requested or required from an operator and/or crewmember. The type of response can be tailored for the severity of the operator's determined state, or the severity of a potential result of operator inaction, such as an impending collision. Further, a frequency or intensity of the alert can increase as time passes without an operator response, and/or the vehicle approaches an imminent hazard.

In a situation where the operator receiving the alert is unable to provide the needed response, the system 100 can control one or more systems to mitigate and/or avoid the upcoming hazard, such as via the command system 108b. The control can be directed to a function of the vehicle itself (*e.g*., activating the breaks), at a system along the roadway/railway (*e*.*g*., activate a track switch to change the path of the vehicle), another vehicle system (*e.g*., an automated response to another vehicle along the roadway/railway), or a combination thereof.

***Human-Machine Interface (HMI) System 104.*** The HMI system 104 provides a control and communication interface for the operator (*e*.*g*., a human operator, whether on-board the vehicle or remote). The HMI system 104 may include a human-machine interface 104, which may be based on a touch screen graphical user interface ("GUI") and/or speech-recognition systems. The human-machine interface 104 may employ, for example, a tablet computer, a laptop computer, a smart phone, or combination thereof. The human-machine interface 104 can be secured near the operator depending on operator preferences. The human-machine interface 104 may be removably coupled to the vehicle cabin or, in certain aspect, employ an integrated display within the cabin (*e*.*g*., an existing display).

The HMI system 104 serves as a channel of communication between the operator and the operator monitoring system 100, enabling the operator to command tasks to and receive feedback and/or instructions from the operator monitoring system 100, to change the allocation of tasks between operator and operator monitoring system 100, and to select which operational applications 202 are currently enabled for the operator monitoring system 100.

As illustrated in Figure 1b, for example, the HMI system 104 may receive status information from a subsystem via the core platform 102, while sending to the core platform 102 mode commands generated by the HMI system 104 or input by the operator. The operator may be remote (*e.g*., on the ground or in another vehicle) or on-board (*i.e*., in the vehicle). Thus, in certain aspects, the HMI system 104 may be remotely facilitated over a network via communication system 122.

As described herein, each of the plurality of subsystems of the operator monitoring system 100 may be modular, such that the entire operator monitoring system 100 can be substantially ported to another vehicle rapidly. For example, the various subsystems may be removably and communicatively coupled to one another via the core platform 102 using one or more software and/or hardware interfaces 220. In certain aspects, however, the operator monitoring system 100 may alternatively be integrated with other vehicle systems, thereby directly employing all sensors and indicators in the vehicle. For example, the operator monitoring system 100, or components thereof, may be integrated into the vehicle during its design and manufacturing.

As illustrated, the core platform 102 may communicate with the other subsystems via one or more software and/or hardware interfaces, which may be a combination of hardware (*e*.*g*., permanent or removable connectors) and software. The core platform 102 can host various software processes that track the operator and vehicle states, as well as any modules for trend analytics (predictive warnings) and machine learning routines. In certain aspects, the operator monitoring system 100 and/or core platform 102 may employ a computer bus and specification

(*e*.*g*., as an interface) that facilitates discovery of a hardware component of a subsystem within the operator monitoring system 100 without the need for physical device configuration or user intervention in resolving resource conflicts. Thus, a user may readily add or remove system or subsystems (*e.g*., as modules) to the operator monitoring system 100 via the core platform 102 without requiring substantial modification and/or integration efforts.

The core platform 102 outputs may be used to provide messages to the HMI system 104. The messages may indicate, for example, checklist progress, contingencies to initiate, warnings to raise, etc. The core platform 102 may also contain a vehicle data recorder, for instance to provide performance review capabilities. The hardware and various computers may also be ruggedized and share a housing with other devices, such as the perception computer. In some examples, the core platform 102 is operatively coupled with a global positioning system ("GPS")/inertial navigation system ("INS") system and power management system. The core platform 102 may also contain a vehicle data recorder, for instance to provide performance review capabilities.

Figure 2 illustrates an enhanced view of the core platform 102 and information storage system 114, as shown in Figure 1a and 1b. For example, core platform 102 includes a plurality of operational applications 202 to provide instructions, perform calculations, process information, and cooperate with other subsystems to monitor a vehicle operator. A plurality of hardware interfaces 220 is configured to send and/or receive information and/or commands to, for example, the response system 108, a vehicle 90, the HMI 104, and any number of other systems and/or subsystems 232 as are desired.

***Operational Applications 202**.* The core platform 102 may provide the operator monitoring system 100 with a plurality of operational applications 202. Examples of such operational applications 202 might include, without limitation, a procesor 204, an anomaly detection system 206, a memory 208 (e.g., computer readable storage device having a vehicle data structure), a machine learning application 210, and other applications and/or systems to perform the funcitions for the core platform 102.

The anomaly detection application 206 employs machine learning techniques to monitor operator characteristics, vehicle states and/or classify sensor inputs in order to detect the presence of non-normal situations, and to identify whether a situation outside of normal operation is present. The anomaly detection application 206 is configured to compare the sensed information against a set of thresholds defined in the fatigue and health classification systems 116, 117. In some examples, identification of a specific condition or characteristic from the anomaly detection application 206 can trigger a warning to be provided to the operator (*e.g*., a visual or audible alert, via warning 108a) and/or a command to be sent to a vehicle system or subsystem (*e.g*., a breaking command, etc., via command 108b).

In monitoring behaviors and/or characteristics of the operator reveals a departure from expected performance, the operator can be alerted, thereby mitigating or avoiding potential mistakes. If an anomaly is detected, the contingency operation application 234 informs and interacts with the operator via the HMI system 104, and may execute a given procedure(s) to respond to the anomaly (*e*.*g*., generate a warning, provide a command, etc.).

In some examples, monitored characteristics and/or cues can indicate one or both of a fatigue condition or a health condition. For example, the operator's head position and/or movement can be captured via one or more sensors (*e.g*., cameras), with data associated with orientation, movement rate, and particular facial movements used by the classification systems 116, 117. For instance, a change in orientation can indicate fatigue, heart failure, hypoxia, and/or seizure. Head movement rate can indicate fatigue as well as seizure. Facial movements can indicate fatigue, heart failure, and/or seizure.

Eye movement can be particularly useful in classifying the operator's state/condition. For example, the operator's blinking rate can indicate fatigue, heart failure and/or seizure. Eye movement can indicate heart failure and/or seizure. Not just rate, but duration of a blink (*e.g*., time of eye closure) is another indicator of fatigue. The heart rate, captured by an optical sensor, or a worn device configured to capture physiological data, can indicate fatigue, heart failure, hypoxia, and/or seizure. Alternatively, in an aircraft, or in another situation where the vehicle will experience a change in pressure (*e*.*g*., achieving high altitudes or a submersible) hypoxia can result from a change in condition. Similarly, in an aircraft, the flight phase can induce fatigue in an operator. Other environmental conditions that can impact the operator's state can be monitored as well. For example, if the cabin air is contaminated and/or lacks oxygen, the operator may experience a drop in attentiveness similar to fatigue and/or a negative health condition. Further, changes in the environment, such as onset of nightfall, may induce sleepiness in the operator. If the trend analysis suggests the operator may respond to a change in illumination, a warning or other response may be generated.

***Machine Learning Application 210.*** In order to continually update the stored information and learn from historical information, the system via the core platform 102 can implement machine learning techniques to aid in identification and interpretation of the various operator conditions, reactions, characteristics, etc., encountered over time. Machine assisted perception technologies, implemented together with machine learning techniques (e.g., artificial intelligence, "Deep Learning" techniques, etc.) can be used. Machine learning is employed because of the complex and varied decisions that are required in the vehicle operational environment, and as the automated systems receive and analyze information from the various sources (*e.g*., cameras, physiological sensors, vehicle state sensors, etc.).

Machine learning is employed as programming each of the variables associated with the changing environment and behaviors cannot be reasonably stored and correlated. Thus, the machine learning alternative enables the core platform 102 to learn from examples as new information is captured. In other words, even a large database of "if, then, else" rules based on expert knowledge were implemented, a limited set of scenarios that correspond to such examples would be addressed by the system, and reaction to new situations would be difficult or impossible. Such a system, employing information storage system 114 of Figures 1 and 2, can build a store of data (*e*.*g*., physiological database 242, health database 244, movement database 248, vehicle state database 250), to provide robust information to form a comparison with captured data (*e*.*g*., via the information collection system 106), analyze operator condition and/or characteristics, in order to generate warnings and/or commands in response to the comparison.

Machine learning techniques can employ data from training exercises (*e.g*., data collection during a real-world operation, and/or simulation of a real-world operation) to create algorithms tailored to specific scenarios, etc. For example, the use of varying types of sensors can determine which sensors collect the most impactful information, and where such sensors should be located. The viability of the different sensors can be tested under a variety of situations, the data being stored and analyzed to generate a simulated environment similar to that of real-world operation of the vehicle. This base of knowledge can be used as comparison with real-time captured data for determining the proper response, as well as updating stored information.

The real-time monitoring and analysis system described herein is configured to operate without the need to develop specific algorithms for each unique situation and/or vehicles, or variations thereof. The machine learning application 210 aids in trend recognition, providing trend analysis developed using machine learning based on, for example, data, lists, matrices, etc., stored in the information storage system 114. In certain aspects, the machine learning application 210 may supply data, or otherwise trigger, the anomaly detection application 206. For example, if the machine learning application 210 detects an undesirable trend, the trend may be flagged as an anomaly and reported to the anomaly detection application 206.

The data may be derived from a combination of encoded data (*e*.*g*., from manuals, compiled data, historical models, etc.) and data acquired in operation (*e*.*g*., via sensors), which supports off-line machine learning and trend analysis. The data to be encoded may be loaded in various human and/or machine-readable formats (*e.g*., .xml format) to describe the contents of procedures and the flow of tasks both within and between procedures. As illustrated in Figure 1b, for example, the information storage system 114 may receive operational commands from the core platform 102, while sending to the core platform 102 configuration data and status and response information generated by the information storage system 114.

In addition to written information, the operator monitoring system 100 may also codify information based on past events and experience of more experienced operators (*e.g*., from monitoring and storing trend information and analysis). Machine learning enables the knowledge acquisition process to be performed efficiently and quickly.

The system 100 incorporates knowledge gained in the areas of human-machine interaction, neurophysiological measurement, and human-subject testing. In some examples, the areas human factors, sleep and behavioral research, and human-system interaction come into play. Further, implementation of the fatigue characteristic measurement, including selection, acquisition, and interpretation of fatigue characteristics, (*e.g*., eye tracking systems) based in part on models and/or data collected in the areas of sleep research, rail human factors, human measurement, signal processing, biomechanics, and cognitive psychology. Moreover, each operation of the system serves to generate and/or update the information storage system 114, and the design of the intervention system machine learning algorithms, and inform the proper employment of various responses and/or interventions.

The data mean and standard deviation of the data can be made over a period of time. Values can be approximate, such as calculated or estimated (*e.g*., if no detailed calibration has been performed by the sensor). The data acquired by sensors can be used to generate a library of events and responses. Additionally or alternatively, this library can be used to statistically define the performance of the vehicle. In this manner, the sensors can be used to statistically define the vehicle responses by logging response to each determined event. In other words, the system can use acquired data to show the mean and standard deviation of forces applied by the vehicle in subsequent operations. The library can also be used to compare present performance of the vehicle to assess the functionality of the system, as described herein.

***Information Storage System 114.*** The information storage system 114 gathers and/or generates a knowledge base necessary to enable the operator monitoring system 100 to determine operator characteristic information. This includes knowledge of operator physiological information (via physiological database 242), health condition information (via health database 244), movement information (derived from movement database 248), and/or vehicle state information (from vehicle state database 250). Physiological database 242 can include data corresponding to a physiological characteristic of the operator (*e.g*., heart rate, respiratory rate, blood pressure, etc.). The health database 244 can store information regarding characteristics of the operator's health (*e.g*., heart function, onset of stroke, unusual body position, etc.). The movement database 248 contains information associated with expected movement of an operator in normal situations experienced during operation. The movement database 248 can build concurrently with the vehicle state database 250, to coordinate a vehicle stimulus (*e.g*., request for a vehicle control) with an operator response (*e.g*., the bodily action expected to address the stimulus).

In some examples, a library or matrix of values associated with a particular characteristic can be stored in a database accessible to the core platform 102. The database can be integrated with system 100 or remotely located (*e.g*., accessed by a network), etc. The monitored characteristics can be compared against the library of values to validate the operator is alert and operating the vehicle as expected.

In each database, the various data can be stored as a series of values in a library or matrix. For example, the data can be converted from raw data (*e.g*., a captured image) into a series of values corresponding to features of the raw data (*e.g*., to a digital representation of a physical action or shape). The values are stored as a tool for comparison, such that data corresponding to expected values are provided for comparison with measured data from the plurality of sensors in conjunction with the fatigue and health classification systems 116, 117.

In an example, no database exists with stored values for a particular characteristic (e.g., pertaining to a newly measured feature), the core platform 102 can build a matrix in accordance with the information acquired by the sensors during monitoring. In examples, during an operation, such as repeated control of a particular instrument (*e.g*., frequent activation of the braking system), the matrix associated with the system can be updated and refined based on acquired operator movement data. Thus, if acquired information deviates from the values in the matrix, an alert can be sent (via warning system 108a) to an operator or other system (*e.g*., a remote controller) via the warning system 108a, or additional information request (*e.g*., from another sensor) to determine whether a fatigue and/or health condition exists, etc.

Additionally or alternatively, the vehicle state database 250 can be populated and adjusted to a specific vehicle during a knowledge acquisition phase (*e*.*g*., during initial setup) such that it contains all the information necessary to operate the vehicle. For example, when transitioning to a new vehicle, the information storage system 114 may perform predefined activities in order to determine the particular vehicle instruments, performance parameters of the vehicle, and other characteristics of the vehicle. The predefined activities may include, for example: (1) generation of a vehicle system model, which informs the operator monitoring system 100 about which systems are onboard and how they are configured, actuation limits, etc.; (2) procedure and checklist codification, which informs the operator monitoring system 100 how to operate the vehicle in normal and non-normal situations; and (3) an operational state model, which informs the operator monitoring system 100 expected responses and/or actions from an alert operator.

The core platform 102 can combine this information with data from a set of internal state sensors, which also improve redundancy and system robustness, thereby allowing the operator monitoring system 100 to generate an accurate estimate of the vehicle state and system statuses, and to identify deviation from expected behavior and/or state of the vehicle. During vehicle operations, the data structure is dynamically updated with real-time data gathered by, *inter alia*, the operator monitoring system's 100, information collection system 106, the HMI system 104, as well as the operator monitoring systems 100 internal state sensing.

Once the vehicle data structure of memory 208 for a given vehicle is populated, the vehicle data structure of memory 208 can then be retained in a vehicle library and used for all other vehicle of the same make and model for which operator monitoring system 100 is available. The vehicle data structure of memory 208 may be further refined as additional data is generated and/or collected by the operator monitoring system 100.

***Hardware Interfaces 220**.* Various information pertaining to the operational applications 202 are communicated between the warning system 108a, command system 108b, vehicle 90, HMI system 104, and other subsystems 232 via, for example, the actuation system 222 (*e.g*., a primary actuation system), actuation system 224 (*e.g*., a secondary actuation system), vehicle operations system 226, HMI system 228, and other interface 230. The hardware interfaces 220 are configured to cooperate with operational applications 202 to communicate with various systems (either directly or via communication system 122).

***Response System 108.*** Response system 108 executes the actions commanded via the core platform 102. As illustrated in Figure 1b, for example, the response system 108 may receive actuation commands and configuration data from the core platform 102, while sending to the core platform 102 status and response information generated by the response system 108. In order to respond to a potential fatigued operator, the operator monitoring system 100 may employ a warning system 108a, while further employing a command system 108b to physically control vehicle systems.

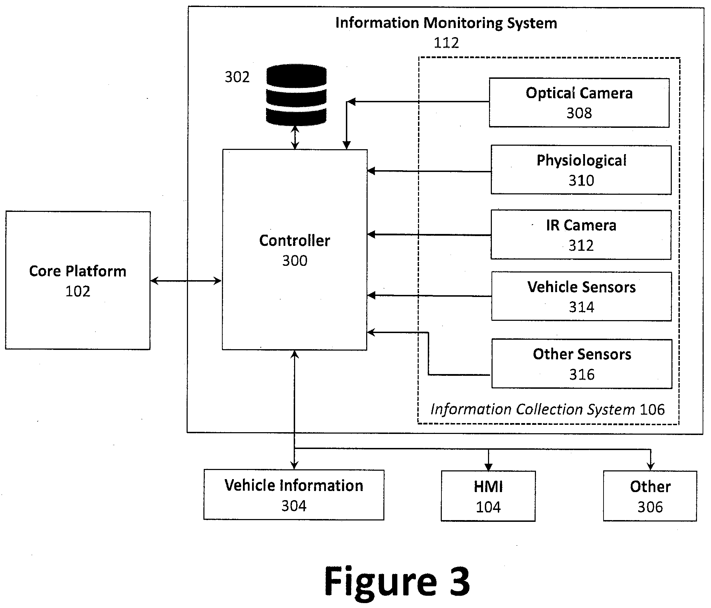

The sensors (*e.g*., cameras) allow for imaging the operator's movements, expressions, vehicle and/or instrument interactions, the operator environment, etc., from a variety of locations and from multiple perspectives. In some examples, sensors can view surfaces and instruments within the vehicle, to capture information regarding the operator's condition, or as a redundant source of information. The various sensors are described with respect to Figure 3, *infra*.

***Human-Machine Interface 104.*** The operator's human-machine interface 104 may employ a tablet based GUI and a speech-recognition interface that enables vocal communications. An objective of the human-machine interface 104 is to enable the operator to interact with the core platform 102's knowledge base in manner akin to the way an operator interacts with a human engineer or crew.

The human-machine interface 104 can display the current state of operator monitoring system 100 (its current settings and responsibilities) as well as which operational applications 202 are currently installed, which operational applications are running and, if they are active, which actions the operational applications 202 are taking. The human-machine interface 104's GUI display may also be night-vision goggles such that information is visible regardless of the operator's eyewear, available lighting. The speech-recognition system may be used to replicate the same types of verbal communications used by human operating crews when running through checklists and communicating on the vehicle. In certain aspects, the speech recognition may be limited to the same standards of codified communications used by operator teams to minimize the chances of the system failing to recognize commands or changing into inappropriate modes of operations. The speech-recognition system may be configured to learn/recognize the speech of a given operator through a voice training protocol. For example, the operator may speak a predetermined script such that the speech-recognition system can become trained with the operator's dialect.

The human-machine interface 104 may provide the status and/or details of various operations, including the entire operator monitoring system 100, the information collection system 106 via a perception status application, autopilot (where applicable), the GPS/INS system, and any other application or system status information (*e*.*g*., via information storage system 114). The display of the human-machine interface 104 may be customized by the operator. For example, the operator may wish to add, reorganize, or remove certain of the display icons and/or operational applications 202, which may be accomplished through a select and drag maneuver. The human-machine interface 104 may further inform the operator regarding the vehicle's operating status and to provide the operator with instructions or advice.

The various operational conditions of the vehicle, which may be gathered from the information collection system 106 or another sensor, may be displayed as alphanumeric characters or as graphical dials (*e.g*., in accordance with the operator's preference settings).

The HMI system 104 may provide an intuitive display and interface that includes checklist verification and alerts from the core platform 102. Thus, the operator may review and monitor checklist items, as well as review any available alerts. Indeed, a function of the HMI system 104 is to facilitate checklist monitoring and/or execution, marking items as complete when the when the information collection system 106 perceives their completion and providing warnings to the operator when items are not completed, as based on information previously imported from, for example, an operator's handbook or operations manual. The operator monitoring system 100 also monitors system status, comparing the current system state to that expected based on the handbook and other knowledge sources, and guides appropriate responses to particular situations.

The HMI system 104 can enable the operator to limit the activities executed by the operator monitoring system 100, if any. Thus, the operator monitoring system 100 may operate, depending on configuration, in an advisory role (i.e., without control over the vehicle), a fully autonomous role (i.e., controlling the vehicle controls without operator intervention), or an advisory role with the ability to control vehicle controllers.

A risk when employing any automation system is the potential for mode confusion on the part of the operator (*e*.*g*., where the operator neglects a task believing that the automation system will handle the task). The human-machine interface 104 may display the information necessary to ensure that the operator is always aware of the mode in which operator monitoring system 100 is operating. Additionally, the HMI system 104 serves as the human interface for individual vehicle applications (*e.g*., operational applications 202).

***Information Monitoring System 112**.* A described herein, and as shown in Figure 3, the information monitoring system 112 collects, determines, or otherwise perceives the real-time characteristics of the operator. As noted above, the information monitoring system 112 maintains a direct connection (*e*.*g*., integral with or otherwise hardwired) to the core platform. As shown in Figure 3, for example, when information collection system 106 is used, the information monitoring system 112 may include a dedicated controller (*e*.*g*., processor) or share a controller (*e.g*., controller 300) of the information collection system 106. Each data type associated with a specific sensor may use a data processing component to reduce noise and eliminate unnecessary artifacts for data processing.

The information collection system 106, for example, may employ a combination of sensors, including, for example, an optical camera 308, a physiological sensor 310, an IR camera 312, a vehicle sensor 314, and any number of alternative and additional sensors 316, for example, audio recording/voice transcription, electroencephalogram (EEG), electrocardiogram (ECG), functional Near Infrared Spectroscopy (fNIRS), respiration, sweat, laryngeal/face/body electromyography (EMG), electrooculography (EOG), externally-facing perception units, temperature sensors, positional sensors, inertial sensors, body weight sensors, accelerometers, blood gas sensors, fluid chemical analysis sensors, etc. Data capture, data fusion, and/or recognition algorithms may be stored in a database (*e.g*., database 302) to aid in determination of one or more operator characterizes, via one or more sensor inputs, including from interaction with vehicle instruments 304, input via an HMI 104, or via other means 306.

Although monitoring eye movement is a useful characteristic for determining fatigue in an operator, eye trackers are not universally suited for use in a vehicle cab environment. Many sensors in use are vulnerable to occlusion effects, such as from eyewear, and are sensitive to head movement. Alternatively, head-mounted eye trackers are cumbersome to wear, and may cause head strain over prolonged periods of wear. Such systems may require calibration to individual users, and may be susceptible to error if a physical vibration or quick or unexpected movement is detected. By contrast, to previous systems, the presently described operator monitoring system captures data corresponding to a plurality of physiological, biological, behavioral, and or health characteristics to identify and/or classify operator fatigue.

Head and body dynamics are used to determine fatigue characteristics. For instance, head drooping and off-axis body positions (*e.g*. off-center posture, reclining, slumped shoulders) typically occur at the onset of sleepiness. Fatigued operators may lean against the control stand and/or prop up one's head with an arm. Prolonged lateral gazing, especially in a forward-facing posture as is typical in vehicle operations, is yet another indicator of lost vigilance and growing fatigue. Additionally or alternatively, sensors such as cameras are used to track the operator's head by identifying body characteristics, such as the nose and mouth, and monitor changes in shape, movement, etc.

Cab activity, or human-machine interaction (*e.g*., interaction with vehicle controls), provides additional or alternative insight as to the operator's performance, which correlates with fatigue. Such interactions can be directly measured by connecting to any existing cab data buses, and/or indirectly measured using sensors (*e.g*., cameras 308, 312, and/or sensors 316) that passively monitor the state of switches, gauges, throttles, etc.

Vision-based cab/cockpit monitoring systems use a plurality of cameras (*e.g*., cameras 308, 312) in a variety of different settings (*e.g*., vehicle types, operational conditions, etc.). Camera systems are designed to sync with direct data connections to accurately determine instrument types (*e.g*., the use of analog versus digital displays), differing lighting conditions, and/or confirm the accuracy of the collected data by employing redundant collection modes. In view of the collected and analyzed data, operator performance is gauged by a comparison of the measured data against a library of stored data corresponding to expected performance values. In this manner, poor performance can be identified by inappropriate system interaction or delayed reaction times.

Output from the information collection system 106 can be used to inform electronic checklists, moving maps, heads-up displays, text-to-speech reminders, etc. Sensor data associated with operator movement is used as indicators of activity, providing a layer of redundancy if the cameras cannot view the control panel due to occlusion.

The data gathered by the information collection system 106 may be encoded and provided to the core platform 102 in real-time. The open architecture of the core platform 102 enables the incorporation of additional data received from the vehicle operating system (*e.g*., via a data bus) to augment the operator characteristic data generated by the information collection system 106. As illustrated in Figure 1b, for example, the information monitoring system 112 and/or the information collection system 106 may receive commands and configuration data from the core platform 102, while sending to the core platform 102 status and vehicle situation information (*e.g*., via a library or matrix stored in vehicle state database 250), data from the information collection system 106, and/or otherwise collected by the information monitoring system 112. Thus, sensors associated with the information monitoring system 112 can be directly linked to the core platform 102, and/or use redundant systems (i.e. visual capture of digital readouts, etc.) to identify elements of the vehicle state and make determinations based thereon.

The operator monitoring system 100 furthers the safety and utility of commercial operations while providing significant savings in human operating costs. For example, the operator monitoring system 100 may be applied to long-haul cargo carriers to increase safety and efficiency as well the cost-savings of this advanced operator-assist technology. Further, the operator monitoring system may serve as a training tool for operators during vehicle operation, or as a safety system, providing a second set of eyes in what would traditionally be a single-operator vehicle. Portions of the HMI 104 streamline all vehicle operations, even multi-crew operations.

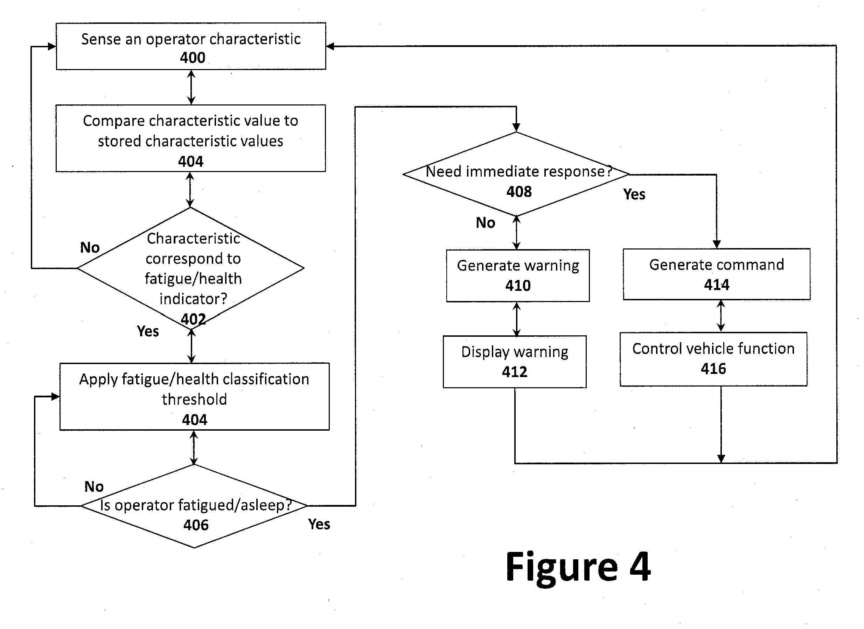

Figure 4 represents a flowchart for an example implementation for an operator monitoring system, in accordance with the present disclosure. As described herein, loss of situational awareness due to fatigue, boredom, and distraction in the locomotive cab are significant problems. Conventional systems aim to mitigate these issues, but there are several drawbacks that do not provide complete, local situational awareness. The currently described operator monitoring system is an automated system that provides real-time sense, analysis, and interaction with the operator, thereby reducing the risk of accident due to fatigue. The operator monitoring system is capable of observing the operator during operation of the vehicle, determining a potential fatigued state, warning or otherwise alerting the operator, to restore focus to the task at hand, giving the operator time to react to hazards and potentially stop the vehicle before a collision can occur.

In an example method 400 of implementing the described operator monitoring system (*e*.*g*., operator monitoring system 100), feedback regarding the operator condition (*e*.*g*., one or more operator characteristics, such as body and eye movement, physiological characteristics, etc.) is determined and employed to mitigate potentially problematic situations.

In an example illustrated in Figure 4, an operator characteristic is sensed/measured via a plurality of sensors (*e.g*., via information collection system 106) in block 402. In block 404, a value associated with the operator characteristic is compared against one or more stored value associated with known, learned and/or calculated operator characteristics (*e.g*., via information storage system 114). In block 402, the comparison is analyzed via one or more classification systems (*e.g*., fatigue classification system 116 and/or health classification system 117) to determine whether the characteristic corresponds to a fatigue and/or health indicator. For example, if the operator is experiencing a condition and/or state that impairs the operator's focus (*e.g*., sleepiness, health emergency), the system is capable of responding with an appropriate response.

If no indicator is determined, the method returns to block 400, and continues to monitor the operator's characteristics. If, however, a fatigue and/or health indicator is determined, the process continues to block 404, where one or more thresholds are applied to the indicator to determine the severity of the operator's condition. At block 406, the core platform 102 determines whether the operator is fatigued, asleep, or otherwise unalert. If there is no such determination, the method returns to block 404, to continue to monitor the operator's condition. If the operator is determined to be fatigued, etc., the method proceeds to block 408, where information regarding the vehicle is considered, as well as the severity of the operator's condition. For example, if the vehicle is in normal operation, and the level of severity of the operator's condition is low, the system may generate a warning (*e.g*., via warning system 108a) in block 410. For instance, a visual and/or audible alert can be provided to the operator in block 412, to refocus the operator's attention.

If, however, the vehicle is moving at a high rate of speed, or operating in a congested area, or otherwise at danger from operator inattention (*e.g*., in flight), the system generates a command (*e.g*., via command system 108b) in block 414. Thus, the command system 108b controls one or more vehicle functions (*e.g*., a braking system) in response to the command, as shown in block 416. In each case, the method would continue to monitor the operator characteristics.

The above-cited patents and patent publications are hereby incorporated by reference in their entirety. Although various embodiments have been described with reference to a particular arrangement of parts, features, and like, these are not intended to exhaust all possible arrangements or features, and indeed many other embodiments, modifications, and variations may be ascertainable to those of skill in the art. Thus, it is to be understood that the invention may therefore be practiced otherwise than as specifically described above.

## Claims

1. A system to monitor an operator of a vehicle (90), the system comprising:
a sensor (316) to collect information regarding one or more characteristics of the operator during operation of the vehicle (90);
a core platform (102) configured to determine whether the one or more characteristics corresponds to a fatigue indicator;
a response system (108) configured to generate a warning (108a) based at least in part on the fatigue indicator; and
an interface (230) to present the warning (108a) to the operator.

2. The system of claim 1, wherein the characteristic corresponds to a physiological characteristic, the sensor (316) comprising a physiological sensor (316) to measure the physiological characteristic.

3. The system of claim 2, wherein the physiological characteristic is one of a heart rate, a respiratory rate, a blood oxygen level, and a body temperature.

4. The system of claim 2 or 3, further comprising a library of physiological characteristic values, wherein the change is determined by a comparison of a measured physiological characteristic value against a corresponding stored physiological characteristic value.

5. The system of claim 4, further comprising a classification system (116, 117) to identify an operator condition based at least in part on the comparison, the measured physiological characteristic value, and the stored physiological characteristic value.

6. The system of claim 5, wherein the classification system (116, 117) comprises one or more thresholds corresponding to the operator condition, wherein the operator condition includes awake, fatigued, and asleep.

7. The system of any previous claim wherein the characteristic corresponds to at least one of (1) a change in head position or orientation, (2) a delayed reaction time, (3) a facial movement, or (4) a change in body position or orientation.

8. A method of monitoring an operator of a vehicle (90), the method comprising:
sensing, via a plurality of sensors (316), one or more characteristics of the operator;
determining, by a core platform (102), whether the one or more characteristics corresponds to a fatigue indicator;
generating, by a response system (108), a warning (108a) based at least in part on the fatigue indicator; and
presenting the warning (108a) to the operator via an interface (230).

9. The method of claim 8, further comprising the step of identifying, by a classification system (116, 117), an operator condition based at least in part on the measured physiological characteristic value, and the stored physiological characteristic value.

10. The method of claim 9, further comprising the step of applying, via the classification system (116, 117), one or more thresholds corresponding to the operator condition.

11. The method of claim 9 or claim 10, wherein the operator condition is at least one of awake, fatigued, or asleep.

12. The method of any of claims 8 to 11, further comprising the steps of:
determining, via the classification system (116, 117), that the operator condition corresponds to being asleep;
generating, via a command system, a command to control one or more vehicle (90) functions in response to the asleep determination; and
controlling one or more vehicle (90) functions in response to the command.

13. The method of any of claims 8 to 12 wherein the one or more characteristics correspond to a physiological characteristic, the plurality of sensors (316) comprising a physiological sensor (316) to measure the physiological characteristic.

14. The method of claim 13, further comprising the steps of:
comparing a measured physiological characteristic value against a corresponding stored physiological characteristic value;
applying one or more thresholds to the comparison; and
determining an operator condition based at least in part on the comparison, wherein the operator condition is at least one of awake, fatigued, or asleep.

15. The method of claim 13, or claim 14 further comprising the step of assuming control or adjusting an operation of the vehicle (90) based at least in part on the fatigue indicator.
